# EUROPEAN PATENT APPLICATION

(11) **EP 1 281 364 A2**
(43) Date of publication of application: **05.02.2003**
(21) Application number: 02016932.2
(22) Date of filing: 31.07.2002
(51) Int. Cl.: A61B 17/70

(54) **Bone connector**

(30) Priority: 01.08.2001 JP 2001233510
(71) Applicant: Showa IKA Kohgyo Co., Ltd., Aichi-ken (JP)
(72) Inventor: Shirado, Osamu, Sapporo-shi, Hokkaido (JP); Oribe, Kazuya, c/o Showa Ika Kohgyo Co., Ltd., Tokyo (JP); Takamido, Hiroshi, c/o Showa Ika Kohgyo Co., Ltd., Nagoya-shi, Aichi-ken (JP)
(74) Representative: Grünecker, Kinkeldey, Stockmair & Schwanhäusser Anwaltssozietät

(57) **Abstract**

A bone connector includes a first screw (5), a second screw (6) and a coil spring (1). The first screw (5) is screwed into one centrum (3). The second screw (6) is screwed into another centrum (4). The coil spring (1) connects an upper portion of the first screw (5) to an upper portion of the second screw (6). The bone connector allows that a positional relation originally set between the centrums (3,4) is changed in some degree.

## Description

### BACKGROUND OF THE INVENTION

### 1. Field of the Invention

The present invention relates to a bone connector which connects bones, and more particularly, to a bone connector having a resilient rod member.

### 2. Description of the Related Art

When connecting bones such as vertebras, the following method has been conventionally employed. A first screw of which a first rod engaging portion is provided on a head portion is screwed into one vertebra. A second screw of which a second rod engaging portion is provided on a head portion is screwed into another vertebra. Since, by means of detent screws or the likes, end portions of a rod member are fixed to the first rod engaging portion and the second rod engaging portion respectively, the rod member is integrally fixed to two screws and the bone connector is formed.

A rigid body has been employed as the conventional rod member, and end portions of the rigid rod member are fixed to two screws respectively.

This rigid rod member is effective when mutual positional relation previously set between one vertebra and another one is constantly fixed and held. However, further improvement is required for permitting free motion of vertebra in some degree while constantly fixing and holding the mutual positional relation previously set between the vertebras.

### SUMMARY OF THE INVENTION

The present invention has been proposed in view of the above-described circumstances, and it is an object of the present invention to provide a bone connector which allows that a positional relation originally set between vertebras is changed in some degree, but after the positional relation is changed, the position is again returned to the originally set positional relation.

In order to achieve the above object, the present invention provides a bone connector comprising: a first fixing member having a lower portion thereof fixed to one bone; a second fixing member having a lower portion thereof fixed to another bone; and a resilient rod member which connects an upper portion of the first fixing member to an upper portion of the second fixing member.

According to this invention, the lower portion of the first fixing member is fixed to one bone, the lower portion of the second fixing member is fixed to another bone, and the resilient rod member connects the upper portion of the first fixing member to the upper portion of the second fixing member. Therefore, the bone connector allows that a positional relation originally set between vertebras is changed in some degree, but after the positional relation is changed, the position is again returned to the originally set positional relation.

In a preferred embodiment of the present invention, the resilient rod member is a coil spring.

In this embodiment, even if the resilient member is the coil spring, the bone connector allows that a positional relation originally set between vertebras is changed in some degree, but after the positional relation is changed, the position is again returned to the originally set positional relation.

In a preferred embodiment of the present invention, the coil spring is wound tight and each convolution of the coil spring is in full contact with neighbor convolutions.

In this embodiment, even if the coil spring is wound tight and each convolution of the coil spring is in full contact with neighbor convolutions, it is realized to provide the bone connector having resilient for fixing a positional relation between vertebras. Further, the bone connector allows that a positional relation originally set between vertebras is changed in some degree, but after the positional relation is changed, the position is again returned to the originally set positional relation.

### BRIEF DESCRIPTION OF THE DRAWINGS

Fig. 1 is a diagram showing one example of a state in which a bone connector connects one vertebra to another one;
Fig. 2 is a diagram showing a state in which two nut members connect end portions of a rod member to two screws respectively;
Fig. 3 is a diagram of the nut member; and
Fig. 4 is a diagram showing one using example of the bone connector.

### DESCRIPTION OF THE PREFERRED EMBODIMENT

An embodiment of a bone connector according to the present invention will be explained in detail based on the drawings.

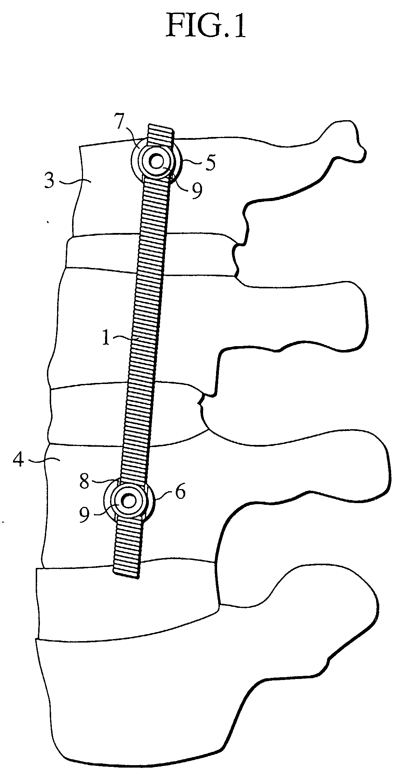

As shown in Fig. 1, a first screw (implant) 5 of which a first engaging groove 7 is provided on an upper (a head) portion is screwed into one centrum (vertebra) 3, a second screw 6 of which a second engaging groove 8 is provided on an upper (a head) portion is screwed into another centrum (vertebra) 4, and end portions of a rod member 1 are fixed to the first engaging groove 7 and the second engaging groove 8 respectively. Therefore, the rod member 1 connects integrally the first screw 5 and the second screw 6 by means of detent screws 9, and the bone connector, for fixing and holding a positional relation between the centrum 3 and the centrum 4, is formed.

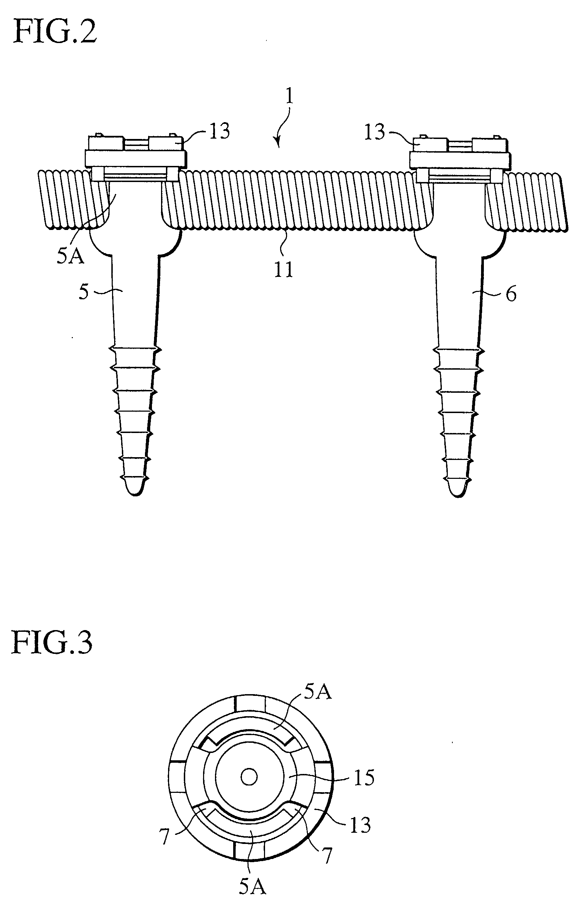

Since the rod member 1 comprises a resilient member which can resiliently be deformed, it allows that the positional relation originally set between vertebras is changed in some degree. In this embodiment, a coil spring 11, which is wound tight and whose convolution is in full contact with neighbor convolutions, to realize a bone connector having a resilient rod member for fixing a positional relation between vertebras (see Fig. 2), is used as the resilient member. The coil spring 11 has a penetrated hole in a hollow portion of it, therefore, bodily fluid does not gather therein. If the hole is not necessary, appropriate resin may be charged into the hollow portion.

Fig. 2 shows that two nut members 13 fix end portions of the rod member 1 to the first screw 5 and the second screw 6 respectively. Fig.1 shows that two detent screws 9 are screwed into the head portion of the first screw 5 and the head portion of the second screw 6 respectively. Fig.2 shows that two nut members 13 are screwed on outside of the head portion of the first screw 5 and outside of the head portion of the second screw 6 respectively, and the rod member 1 is fixed to both of the first screw 5 and the second screw 6.

Fig.3 shows a diagram of the nut member 13 which is screwed on outside of the head portion of the first screw 5. Also, the nut member 13 screwed on outside of the head portion of the second screw 6 has the same conformation. In this case, as shown in Fig. 3, the nut member 13 is screwed on a male thread which is provided on an outer peripheral surface of two rising portions 5A formed at the head portion of the first screw 5, and the rising portions 5A is disposed to be opposed each other. According to such a conformation, the rising portions 5A can be prevented from separating from each other. A thin and long pushing tool 15, which can be rotated, pushes the rod member 1 engaged with the engaging groove 7 of the first screw 5. In the above configuration, it is possible to reliably fix and hold the rod member 1 to the first screw 5.

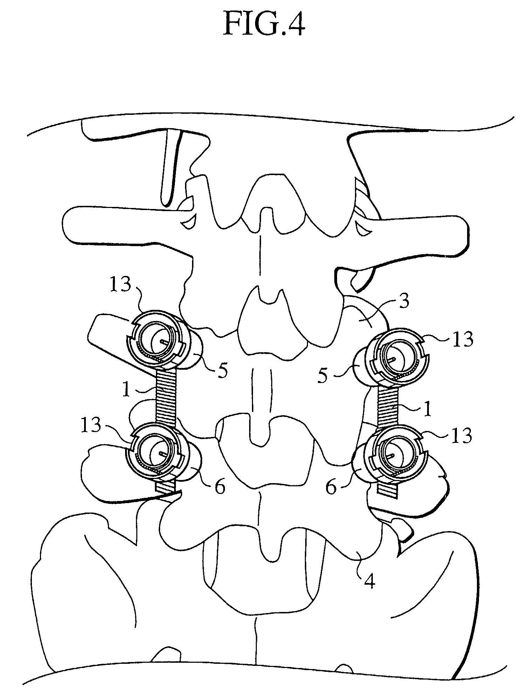

Fig.4 shows one using example of the bone connector. The first screw 5 is screwed into centrum 3, the second screw 6 is screwed into cetrum 4, and the end portions of the rod member 1 are fixed to the head portion of the first screw 5 and the head portion of the second screw 6 respectively. Thereby holding the positional relation between the centrum 3 and the centrum 4. With this design, the positional relation between two centrums is constantly held in the fixed state, and displacement of the two centrums in their approaching direction is limited. Also, the rod member 1 is resiliently deformed because it is comprised a resilient member. Therefore, the bone connector having the resilient rod member 1 allows to do forward inclination, backward inclination, twisting of body and motion of centrums in their separating direction which cause change of the positional relation between the two centrums.

Also, the rod member 1 functions so as to return to the original state after the resilient deformation. Therefore, the originally set positional relation of the centrums is maintained.

Thus, the rod member 1 has a feature that it is prone to be resiliently deformed and is prone to return to its original state, because the rod member 1 comprises a coil spring 11 which is wound tight. Therefore, the bone connector having the resilient rod member is allowed to move in some degree according to patient motion for a short period of time and to maintain the originally set positional relation of the centrums for a long period of time.

The above-described embodiment is one example of the present invention. Thus, the present invention is not limited to the above embodiment, and various modifications can be made in accordance with configuration within a range not departing from technical idea of the invention.

That is, one example of the coil spring, which is wound tight, is explained in the above embodiment, but the present invention is not limited to this. The coil spring may be of tapered shape, a central portion of the coil spring in its longitudinal direction may have a diameter greater or smaller than its ends. Further, instead of wounding the coil tight, the coil spring may be wound at appropriate pitch.

## Claims

1. A bone connector comprising:
a first fixing member (5) having a lower portion thereof fixed to one bone (3);
a second fixing member (6) having a lower portion thereof fixed to another bone (4); and
a resilient rod member (1) which connects an upper portion of the first fixing member (5) to an upper portion of the second fixing member (6).

2. A bone connector according to claim 1, wherein the resilient rod member (1) is a coil spring (11).

3. A bone connector according to claim 1, wherein the coil spring (11) is wound tight and each convolution of the coil spring is in full contact with neighbor convolutions.
